# FASCICULE DE BREVET EUROPEEN

(11) **EP 3 247 504 B1**
(45) Date de publication et mention de la délivrance du brevet: **19.12.2018**
(21) Numéro de dépôt: 15707044.2
(22) Date de dépôt: 23.01.2015
(51) Int. Cl.: B05B 11/04, B05B 7/24, A61M 11/06, A61M 11/02

(54) **DISPOSITIF DE DÉLIVRANCE D'UN PRODUIT PAR PULVÉRISATION**
VORRICHTUNG ZUR AUSGABE EINES PRODUKTES DURCH SPRÜHEN
DEVICE FOR DISPENSING A PRODUCT BY SPRAYING

(43) Date de publication de la demande: 29.11.2017
(73) Titulaire: Gerbron, Jacques, 06500 Menton (FR); Lagneaux, Patrick, 59494 Aubry Du Hainaut (FR); Yuan, Jian Jun, Yuyao, Zhejiang 315400 (CN)
(72) Inventeur: Gerbron, Jacques, 06500 Menton (FR); Lagneaux, Patrick, 59494 Aubry Du Hainaut (FR); Yuan, Jian Jun, Yuyao, Zhejiang 315400 (CN)
(74) Mandataire: Decobert, Jean-Pascal
(86) Numéro de dépôt international: PCT/EP2015/051303
(87) Numéro de publication internationale: WO 2016/116163

(56) Documents cités:
- FR-A- 1 164 124
- FR-A1- 2 292 525
- FR-A1- 2 354 146
- US-A- 4 245 788

## Description

### DOMAINE DE L'INVENTION

La présente invention concerne un dispositif de délivrance d'un produit par pulvérisation ainsi qu'un pulvérisateur équipé de ce dispositif.

L'invention trouvera particulièrement son application dans le domaine de la délivrance de produits sous forme de fluides ce qui inclut ici les poudres, notamment à usage cosmétique ou encore médical ou vétérinaire.

### ETAT DE LA TECHNIQUE

Dans ce domaine, la publication FR-A-2 918 999 divulgue un distributeur de produits fluides employant la technique de l'effet Venturi de sorte à produire, par l'application d'un jet d'air sous pression, un effet dépressionnaire au niveau d'une embouchure d'un canal de sortie de produit fluide à délivrer. L'opérateur appuie sur la surface extérieure d'une poire déformable de sorte à créer le flux d'air dépressionnaire. Le demandeur a constaté que ce type de pulvérisateur n'était pas exempt d'inconvénients notamment en ce qui concerne l'efficacité de la projection. Plus précisément, un appui relativement important sur la poire ne permet qu'une délivrance relativement faible de produit fluide.

Le document FR-A1-2292525 divulgue une tête de pulvérisateur avec deux conduits imbriqués pour l'aspiration d'un produit depuis un des conduits par dépression d'air issu de l'autre des conduits. Cependant l'efficacité de ce dispositif est perfectible.

Le document FR-A1-2354146 divulgue un dispositif de vaporisation comprenant un premier canal d'amenée d'un produit vers une sortie et un deuxième canal de gaz apte à mettre en dépression la zone de sortie du premier canal. Un élément mobile entre deux positions formant membrane permet d'ouvrir ou de fermer le deuxième canal.

C'est un objet de l'invention que de remédier en tout ou partie aux inconvénients des techniques connues jusqu'à présent.

### RESUME DE L'INVENTION

A cet effet, l'invention présente suivant un premier aspect de modes de réalisation, un dispositif de délivrance d'un produit par pulvérisation, comportant un premier canal de circulation du produit vers une sortie du premier canal et un deuxième canal de circulation d'un gaz destiné à produire une dépression à la sortie du premier canal pour une aspiration du produit par effet Venturi. Le dispositif comporte :
- une membrane configurée pour obstruer le deuxième canal dans une première position et pour ouvrir le deuxième canal dans une deuxième position ;
- un plateau s'étendant transversalement dans le deuxième canal et configuré pour s'appliquer sur une face amont de ladite membrane dans la première position, le plateau comportant au moins une ouverture de passage de gaz.

Dans la deuxième position, la surface d'application de la membrane sur le plateau est réduite, voire nulle, de sorte à ce qu'au moins une ouverture du plateau ne soit plus obstruée par la membrane.

Cet aspect de l'invention permet de créer une valve dont la partie mobile comprend la membrane et dont le plateau forme le support. Un effet de surpression est produit grâce à cette disposition : la membrane ne libère le passage de gaz que lorsqu'elle est mise en mouvement, de sorte que le passage de gaz est brutal, favorisant l'effet Venturi.

Suivant un aspect, est présenté un dispositif de délivrance d'un produit par pulvérisation, comportant un premier canal de circulation du produit vers une sortie du premier canal et un deuxième canal de circulation d'un gaz destiné à produire une dépression à la sortie du premier canal pour une aspiration du produit par effet Venturi. Avantageusement, ce dispositif est caractérisé par le fait que le premier canal et le deuxième canal ont des directions moyennes parallèles au niveau de la sortie du premier canal.

Autrement dit, au niveau de la sortie du premier canal, les flux de gaz (généralement de l'air) et de produit (telle une poudre) suivent des directions moyennes parallèles. Cela permet en particulier de limiter les effets de turbulence au niveau de l'embouchure de sortie, turbulences susceptibles de créer des effets de retour de poudre vers l'intérieur du dispositif et limitant l'efficacité de la projection. En effet, l'effet dépressionnaire se trouve généralement réduit par la turbulence tout comme la vitesse de projection finale.

Un autre aspect de l'invention concerne un pulvérisateur comportant un contenant apte à recevoir un produit à pulvériser, un dispositif et une canule plongeant dans le contenant et raccordée au premier canal.

L'invention est par ailleurs relative à un procédé de pulvérisation.

L'invention est enfin relative à une utilisation d'un pulvérisateur pour la pulvérisation d'un produit sous forme de poudre ou de fluide en général.

### BREVE INTRODUCTION DES FIGURES

Les dessins ci-joints sont donnés à titre d'exemples et ne sont pas limitatifs de l'invention. Ils représentent seulement un mode de réalisation de l'invention et permettront de la comprendre aisément.
La figure 1 est une vue de côté du dispositif de l'invention et la figure 2 en illustre une vue en coupe longitudinale.
La figure 3 est une vue de la face arrière d'un dispositif de l'invention.
La figure 4 montre une vue en perspective.
La figure 5 montre une vue en coupe longitudinale réalisée en perspective.
La figure 6 est une vue en coupe longitudinale d'une partie du dispositif de l'invention.
La figure 7 montre une variante de réalisation de l'invention.

### DESCRIPTION DETAILLEE

Avant d'entrer dans le détail de modes de réalisation de l'invention plus particulièrement en référence aux dessins, on énonce ci-après différents aspects de l'invention :
- le premier canal et le deuxième canal ont des directions moyennes parallèles au niveau de la sortie du premier canal ;
- les directions moyennes sont confondues ;
- le premier canal est défini par la paroi interne d'un conduit entouré par le deuxième canal ;
- le premier canal a une section transversale circulaire et le deuxième canal a une section transversale annulaire;
- le deuxième canal est défini par la paroi externe du conduit d'une part et par la paroi interne d'un corps renfermant le conduit ;
- le conduit comporte une zone de section longitudinale de forme conique dégressive en direction de la sortie ;
- la section transversale du deuxième canal est dégressive en direction de la sortie ;
- le deuxième canal comporte une zone dont la section longitudinale est de forme conique dégressive en direction de la sortie ;
- ladite zone se poursuit en aval de la sortie ;
- le dispositif comporte une membrane configurée pour obstruer le deuxième canal dans une première position et pour ouvrir le deuxième canal dans une deuxième position ;
- la membrane est configurée pour passer de la première position à la deuxième position sous l'exercice d'une pression de gaz d'une valeur supérieure à un seuil prédéfini en amont de la membrane ;
- il comporte un plateau s'étendant entre la paroi externe du conduit vers la paroi interne du corps et configuré pour s'appliquer sur une face amont de la membrane, le plateau comportant au moins une ouverture de passage de gaz ;
- la membrane est solidaire par une bordure extérieure, de la paroi interne du corps et la membrane comporte une ouverture traversée par le conduit
- le corps comporte sur sa paroi interne, une butée configurée pour recevoir en appui une surface aval d'une zone périphérique de la membrane ;
- la membrane est solidaire de la paroi externe du conduit et dans la membrane comporte bordure extérieure mobile entre la première position et la deuxième position;
- la paroi externe du conduit comporte une rainure dans laquelle est montée la bordure d'une ouverture de la membrane.
- le corps comporte, sur sa paroi interne, une butée configurée pour recevoir en appui une surface aval d'une zone périphérique du plateau.
- le conduit et le plateau sont monoblocs ; en particulier, ils peuvent être réalisés d'un seul tenant une seule pièce d'une seule matière.
- la paroi interne comporte une portion filetée en amont de la butée.

La présente invention concerne aussi un pulvérisateur dans lequel le contenant comporte au moins une portion de paroi souple configurée pour appliquer une surpression au contenant lors d'un appui d'un utilisateur sur ladite portion et l'utilisation de ledit pulvérisateur pour la pulvérisation d'un produit sous forme de poudre.

Les différentes figures présentent un dispositif de délivrance d'un produit par pulvérisation. Une application préférée mais non limitative est la pulvérisation de produits pulvérulents tels que des poudres par exemple à usage cosmétique ou médical. Le dispositif de l'invention est apte à être rapporté sur un contenant 26 de sorte à charger le dispositif en produit à délivrer.

D'une façon générale, le contenant pourra être en toute matière et par exemple en matière plastique. Le dispositif et le contenant 26 sont avantageusement reliés pour la circulation de produit par une canule telle une tige creuse plongeant dans le contenant avec une extrémité distale située à proximité d'une partie de fond du contenant et une extrémité proximale raccordée au dispositif de délivrance par un premier canal 10 décrit ci-après plus en détail. L'invention ne fait pas d'hypothèse sur le matériau du contenant 26 qui pourra être par exemple en matière plastique. Néanmoins, un intérêt potentiel de l'invention est de pouvoir exploiter la déformabilité d'une portion au moins de la paroi du contenant 26 de sorte à produire la circulation du gaz dépressionnaire. Plus particulièrement, le dispositif de l'invention permet notamment dans le mode de réalisation illustré aux dessins d'exploiter l'air contenu dans le contenant 26, en particulier au-dessus de la poudre stockée et d'opérer une pression sur la paroi du contenant 26 de sorte à expulser l'air et créer l'effet de dépression Venturi. A cet effet, au moins une partie de la paroi du contenant 26 est avantageusement déformable de manière élastique de sorte qu'un utilisateur peut opérer une pression pour diminuer le volume intérieur du contenant 26 et de sorte que le contenant 26 reprend sa forme initiale lorsque l'utilisateur met fin à son effort de pression. A titre d'exemple, on peut utiliser un contenant 26 dont une paroi latérale est en matière plastique souple et légèrement bombée, la configuration du contenant 26 étant agencée de sorte qu'une flexion sur la paroi du contenant 26 produit l'effet dépressionnaire, cette flexion étant réversible élastiquement.

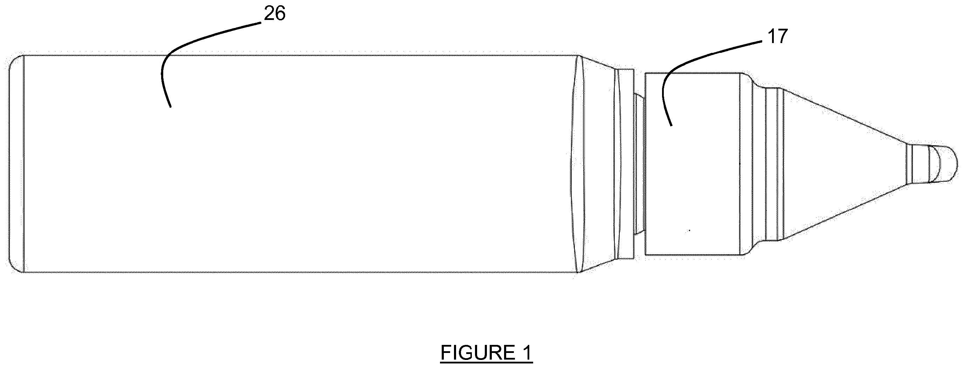

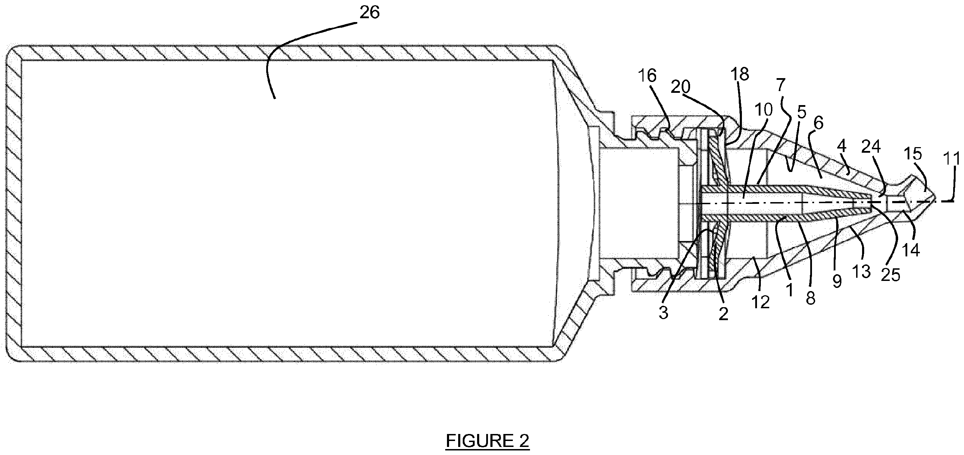

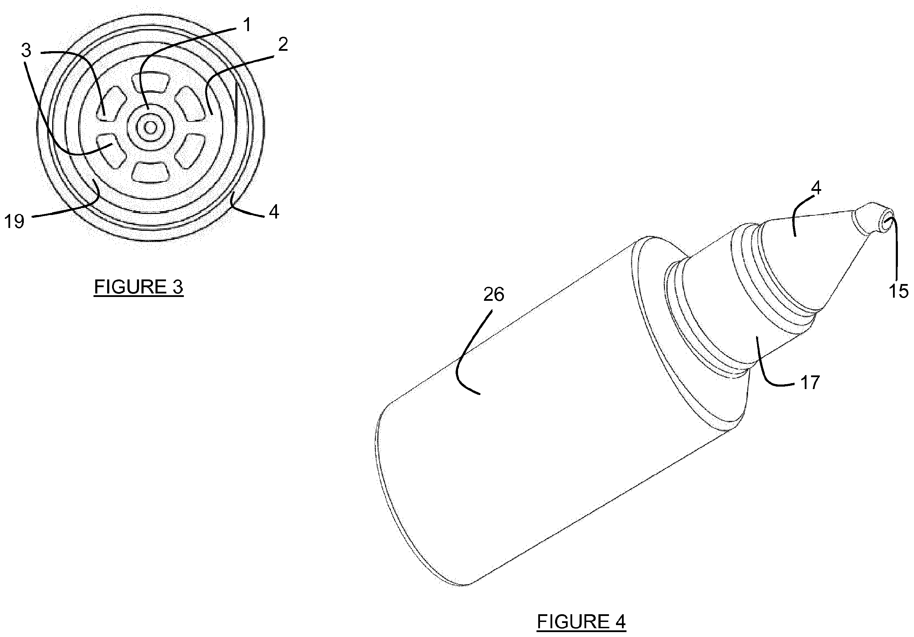

Le mode de réalisation de l'invention est donc plus particulièrement adapté à ce type de génération de flux de gaz dépressionnaire. On remarque à ce titre en figure 2 que l'ensemble des composants du dispositif de l'invention peut être intégré dans un corps 4 dont l'aspect extérieur est notamment visible aux figures 1 et 4, il s'agira généralement d'un corps en matière plastique présentant une première extrémité constitutive d'une zone de raccordement 17 apte à être rapportée sur le contenant 26 et d'une autre extrémité constitutive d'une embouchure 15 au niveau de laquelle le produit à délivrer est évacué. Par exemple, la zone de raccordement 17 peut comporter un filetage 16 de sorte à coopérer avec le goulot d'un contenant lui-même équipé de filets correspondant au niveau de l'embouchure 15. L'invention ne fait pas d'hypothèse sur sa configuration. Dans l'exemple considéré, l'embouchure 15 est une ouverture distale du corps 4, légèrement évasée par rapport à une portion précédente du canal d'évacuation du produit et par ailleurs dans l'exemple incliné relativement à un axe 11 constituant la direction longitudinale générale du dispositif. On peut par exemple ainsi délivrer un produit de manière oblique lorsque le dispositif est situé à la verticale, c'est-à-dire que l'axe 11 est dirigé sensiblement suivant la direction verticale.

A l'intérieur du corps 4, on trouve préférentiellement en premier lieu un premier canal 10 dont la fonction est d'amener le produit vers la portion du dispositif située dans la zone de l'embouchure 15. Plus précisément, le premier canal 10 se termine par une sortie 25, sortie définissant, immédiatement dans sa partie aval, une zone 24 de confluence entre le flux de produit aspiré et le flux de gaz permettant la dépression.

D'une manière générale, dans la présente description, on entend par amont une partie du dispositif située généralement plus en arrière relativement au sens d'écoulement de la poudre et du gaz dépressionnaire. De manière similaire on entend par aval, une partie du dispositif située sensiblement plus vers l'avant suivant la direction de circulation du produit à délivrer et du gaz dépressionnaire.

Avantageusement, le premier canal 10 est placé longitudinalement selon l'axe 11. Ce premier canal 10 peut être défini par la surface interne d'un conduit 1, par exemple sous forme de tube, par exemple en matière plastique, dirigé suivant l'axe. Une extrémité amont du conduit 1 peut coopérer, de manière monobloc ou rapportée, avec une canule constituant l'élément plongeant dans le contenant de sorte à réaliser l'alimentation en produit. Un simple emmanchement peut suffire dans le cas où la canule est rapportée à l'extrémité amont du conduit 1.

A titre préféré, le conduit 1 comporte une première partie depuis son extrémité amont vers la sortie 25 sous forme cylindrique de sorte à délimiter un premier canal 10 de section circulaire constante sur une première partie du conduit 1. Un exemple de portion cylindrique 8 est illustré en figure 2. Ensuite, avantageusement, le conduit 1 comporte une portion conique 9 depuis une section circulaire équivalente à celle de la portion cylindrique 8 vers une section circulaire de diamètre inférieur en direction de la sortie 25. De ce fait, le premier canal 10 adopte dans cette section une section dégressive favorisant l'augmentation de la vitesse de poudre dans le premier canal 10. A titre non limitatif, le premier canal 10 comporte ensuite une portion terminale de section cylindrique jusqu'à l'extrémité au niveau de laquelle se situe la sortie 25.

Pour réaliser l'effet Venturi, le dispositif de l'invention est aussi configuré pour réaliser une circulation d'un gaz sous pression susceptible d'aspirer le produit circulant à l'intérieur du premier canal 10. A cet effet, de manière avantageuse, le dispositif comporte un deuxième canal 6 dont un exemple est aussi présenté en figure 2. D'une manière générale, il est avantageux que le deuxième canal 6 réalise au niveau de la zone de confluence 24, un flux dont la direction moyenne est équivalente à celle de la direction moyenne du flux du produit. Dans le cas représenté, on s'arrange pour que l'ensemble ait une direction dans la zone de confluence 24, parallèle et avantageusement confondue à l'axe 11. Pour y parvenir, la figure 2 illustre un exemple dans lequel le deuxième canal 6 est réalisé de manière symétrique autour de l'axe 11. Dans ce cas, le deuxième canal 6 est défini, dans une partie intérieure, par la paroi externe 7 du premier conduit 1 d'une part. D'autre part, dans sa partie extérieure, le deuxième canal 6 est défini par la paroi interne 5 du corps 4. Le corps 4 comporte en outre avantageusement une portion cylindrique 12 située sensiblement plus en amont d'une portion conique 13 du corps 4 qui est dirigée vers l'avant du dispositif en direction de l'embouchure 15. Ainsi, de manière similaire au premier canal 10, le deuxième canal 6 comporte une première partie dans laquelle l'écoulement s'effectue suivant une section sensiblement cylindrique et une deuxième partie pour laquelle la section est conique. La section conique de la portion 13 présente avantageusement un diamètre de base équivalent à celui de la portion cylindrique 12 et un diamètre final, dans l'exemple légèrement au-delà de la sortie 25, de diamètre sensiblement plus grand (par exemple entre 1,5 et 2 fois le diamètre du premier canal 10 au niveau de la sortie 25). On comprend que le deuxième canal 6 entoure le premier canal 10 tout en suivant une configuration sensiblement similaire, à savoir une première section plus large puis une dégressivité de la section de sorte à accélérer le flux dans une partie conique allant jusqu'à la zone de confluence 24. L'ensemble est avantageusement symétrique autour de l'axe 11 de sorte le premier canal 10 et le deuxième canal 6 sont centrés l'un sur l'autre. Le premier canal 10 étant de section circulaire, le deuxième canal 6 est alors de section annulaire.

Le deuxième canal 6 est agencé de sorte à être en communication avec le volume intérieur du contenant. A cet effet, le raccordement par la zone 17 du dispositif sur le contenant produit cette mise en continuité. On comprend qu'une dépression opérée sur le contenant va produire une circulation d'air entrant depuis la zone de raccordement 17 vers le deuxième canal 6 jusqu'à l'embouchure 15.

Avantageusement, le conduit 1 est maintenu en position par rapport au corps 4 par l'intermédiaire d'un plateau 2, par exemple sous forme d'une pièce de section sensiblement circulaire s'étendant depuis la paroi externe 7 du conduit 1 vers la paroi interne 5 du corps 4. A ce niveau, le plateau 2 peut être maintenu en position par butée sur une butée 18 réalisée sur le corps 4 et visible dans un premier mode de réalisation en figure 2. Plus précisément, dans une étape de raccordement du contenant, la surface supérieure du goulot du contenant peut être appliquée sur une première surface périphérique du plateau 2 alors que cette surface périphérique subit un contre-appui par la butée 18. La butée 18 peut être réalisée par une zone de rétrécissement, par exemple en fond de filets 16.

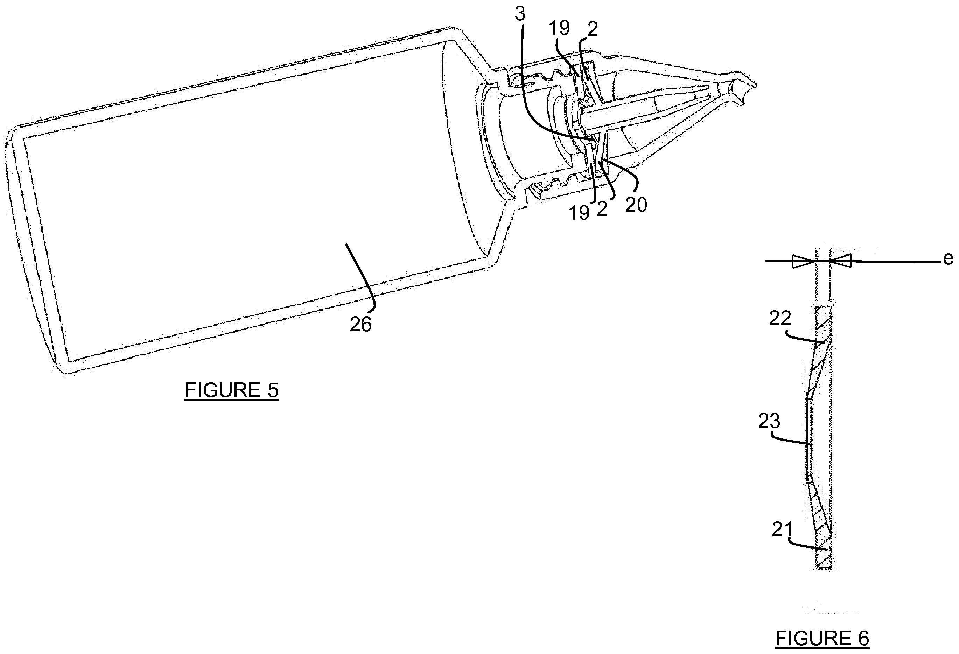

Un joint 19 particulièrement visible en figure 5 peut permettre d'assurer l'étanchéité périphérique de sorte que l'intégralité du flux d'air est orienté vers l'intérieur du corps 4 sans perte au niveau des filets 16. Ce joint peut être porté par le plateau 2 ou par la surface interne du corps 4 ou encore l'une de ces deux dernières pièces peuvent suffire à l'étanchéité. Le plateau 2 comporte une ou plusieurs ouvertures 3 permettant le passage de l'air. Bien entendu, toute autre configuration de plateau ou tout autre type d'armature ou de moyen de fixation susceptible de maintenir le conduit en position à l'intérieur du corps 4 est réalisable sans sortir du cadre de l'invention.

Cependant, le mode de réalisation illustré offre l'avantage de pouvoir coopérer avec une membrane 20 décrite ci-après plus en détail. En effet, on comprend que lors d'une pression de l'utilisateur, l'air est amené rapidement vers la zone de confluence 24 où s'opère l'effet Venturi. Cependant, une telle disposition est fortement dépendante du mode d'appui de l'utilisateur par exemple, les pressions de l'utilisateur peuvent varier en intensité et en vitesse ce qui produit des sorties de produit différentes. Cela peut être nuisible au fonctionnement et notamment à la répétabilité de la délivrance de produit, par exemple dans une quantité déterminée. Pour remédier à cet inconvénient, une membrane 20 est avantageusement positionnée dans le dispositif de sorte à réaliser une fonction de valve. La membrane 20 comporte une première position dans laquelle elle obture le passage d'air depuis la zone de raccordement 17 vers le deuxième canal 6. Dans cette situation, on comprend que le volume intérieur est maintenu étanche et que le dispositif évite ainsi les entrées intempestives, par exemple d'humidité. Par ailleurs, le deuxième canal 6 est clos, c'est-à-dire qu'aucun effet Venturi ne se produit dans cette position d'obturation. La membrane 20 est par ailleurs configurée pour pouvoir adopter une deuxième position dans laquelle elle est déformée de sorte à ouvrir une ou plusieurs des ouvertures 3 réalisées dans le plateau 2. Cette deuxième position est prise par la membrane 20 avantageusement par déformation élastique de sa matière.

Les figures 5 et 6 présentent plus en détail un exemple de formes utilisables pour la membrane 20. Dans le cas de la figure 6, on note que la membrane 20 comporte une zone périphérique 21 susceptible d'être maintenue en position au niveau de la zone périphérique du plateau 2, par exemple par encastrement par l'intermédiaire de la butée 18, de manière similaire à ce qui a été indiqué auparavant. En allant plus vers l'intérieur de la membrane 20, celle-ci comporte une paroi, de section avantageusement dégressive, constituant une zone mobile 22 se terminant jusqu'à un trou 23. Ce trou 23 est configuré pour s'appliquer à proximité de la paroi externe 7 du conduit 1 comme cela est représenté en figure 5. D'une manière générale, la zone mobile 22 est configurée pour recouvrir l'intégralité des ouvertures 3 lorsque le dispositif est en position d'obturation. Par contre, lorsque la membrane 20 est déformée, la partie 22 subit un mouvement, dirigé vers la partie aval du dispositif, et permettant d'ouvrir au moins partiellement l'ouverture 3.

On pourra par exemple utiliser une paroi 22 élastiquement déformable du type matière élastomère ou silicone ou caoutchouc naturel. Ce cas n'est pas limitatif cependant et d'autres dispositions de mobilité peuvent être utilisées pour la paroi 22. Notamment, la membrane 20 pourrait comporter une partie périphérique 21 reliée à la paroi 22 par des moyens mécaniques d'articulation comportant des moyens de rappel en position d'obturation.

A titre d'exemple, l'épaisseur de la paroi périphérique 21 peut être de l'ordre d'un millimètre et celle de la partie distale de la paroi 22 peut être de l'ordre d'un à 3 dixièmes de millimètre. En outre, telle que représentée, la paroi 22 peut être légèrement dirigée vers l'avant (vers la portion aval du dispositif) en allant vers le trou 23. Le plateau 2 est avantageusement configuré de manière similaire. Il existe donc préférentiellement une inclinaison de sorte que le plateau 2 et la membrane 20 sont inclinés vers l'aval du dispositif en se rapprochant de l'axe 11. Cette disposition favorise une déformation en flexion rapidement réversible pour la membrane 20. On comprend que d'une part la membrane peut réaliser une fermeture étanche à l'air du dispositif en position d'inutilisation. En outre, la membrane 20 constitue un organe dont l'effort d'application sur le plateau 2 peut être vaincu par la pression d'air administrée par l'utilisateur pour que le dispositif puisse fonctionner. Ainsi, la présente invention met en pratique un effet de seuil, seuil qu'il convient de dépasser pour que la délivrance du produit intervienne. On comprend de cette façon que des pressions trop légères et inefficaces de l'utilisateur n'auront aucun effet. Par contre, dès que le seuil prédéterminé sera dépassé, la délivrance efficace du produit sera réalisée.

L'appui de la membrane 20 sur le plateau au niveau des trous 3 peut être plan sur plan.

A l'utilisation le dispositif est raccordé au contenant 26, par exemple par l'intermédiaire du filetage 16. Lors de cette étape, simultanément, l'ensemble constitué par le joint éventuel 19, le plateau 2 et la membrane 20 est fixé en position par appui sur la butée 18. Ensuite, lors de l'exercice d'une pression suffisante sur une partie déformable de la paroi du contenant 26, l'utilisateur produit une pression permettant la déformation de la partie mobile de la membrane 20 et l'admission d'un flux d'air à l'intérieur du deuxième canal 6. Ce flux est accéléré par la partie conique du deuxième canal 6 jusqu'à la zone où se situe la sortie de produit au niveau de laquelle les flux d'air et de produit sont en confluence. A ce niveau, le flux d'air issu du deuxième canal 6 produit une aspiration de sorte à favoriser la remontée du produit à l'intérieur du premier canal 10. Le mélange d'air et de produit formé dans la zone de confluence 24 est ensuite évacué par l'embouchure 15. Une portion cylindrique distale 14 peut être disposée entre l'embouchure 15 et la zone de confluence 24 de sorte à stabiliser le flux avant son changement de direction.

On notera que l'invention met en oeuvre peu de pièces et que le montage est facilité du fait que l'on exploite des parties pouvant être solidarisées entre elles et au contenant en une seule étape. En outre, tel que le montre la figure 3, l'ensemble est avantageusement symétrique autour de l'axe 11 de sorte que la fabrication est aussi facilitée. Malgré cette symétrie, le produit peut être projeté suivant une direction différente de l'axe 11, par exemple par une embouchure oblique révélée notamment à la figure 4.

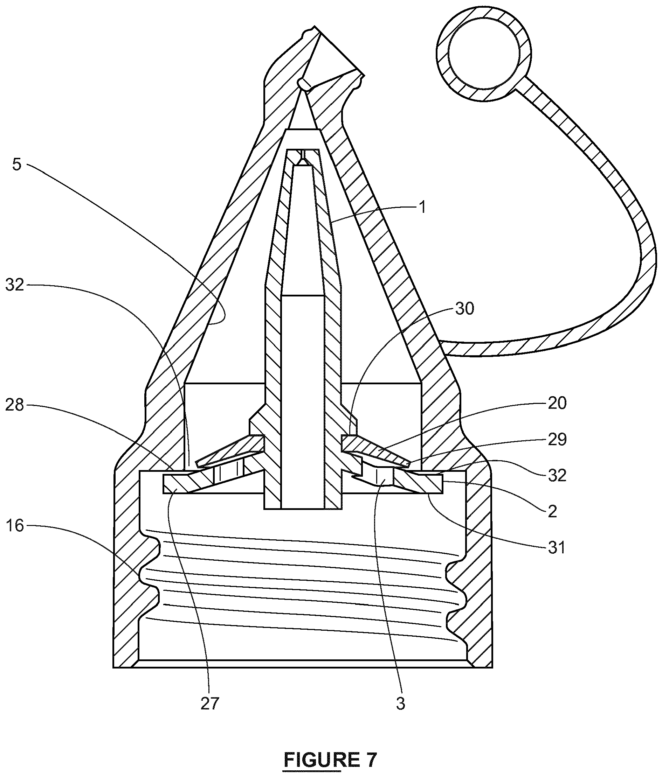

Dans la variante de la figure 7, la membrane 20 est mobile entre la première et la deuxième position par sa périphérie extérieure. A cet effet, la membrane 20 est solidaire de la paroi externe du conduit 1. Cette solidarisation peut par exemple être produite avec une rainure 30 située sur le conduit 1, dans laquelle s'insère la bordure d'une ouverture ménagée dans la membrane 20. La position de repos de cet assemblage est illustrée en figure 7. De préférence, pour faciliter la mise en place de la membrane dans la rainure, le flanc aval de cette dernière est incliné depuis la base de la paroi extérieure du conduit 1 vers le sommet de la rainure, de sorte que le montage de la membrane par son ouverture est opéré progressivement. Une fois en place, cette partie de la membrane n'a plus de déplacement relativement au conduit 1.

La membrane est de préférence de section d'épaisseur décroissante (par exemple linéairement) vers sa périphérie de sorte à augmenter sa déformabilité vers sa bordure extérieure 29 décrite ci-après.

A l'opposé de l'ouverture coopérant avec la rainure 30, la membrane comporte en effet une bordure extérieure 29 libre si bien que la membrane peut se déformer par cet endroit lors de modifications de pression. Un jeu 32 est de préférence préservé entre la paroi interne du corps 4 et la bordure 29. En amont de la membrane 20, le plateau 2 est toujours présent et la coopération de ces deux pièces permet, comme précédemment, la fonction de valve. On notera que l'ensemble conduit 1, plateau 2 et membrane 20 peut être monté d'un seul tenant dans le dispositif ce qui facilite l'assemblage. Cette fois, c'est avantageusement une zone périphérique 27 du plateau 2 qui s'applique sur la paroi interne 5, au niveau d'une butée 28, par une surface aval 32. En contre-appui, une surface amont 31 du plateau 2 peut coopérer avec l'extrémité supérieure d'un contenant, par exemple au niveau d'un goulot doté d'un filetage complémentaire à celui du corps 4. A l'extrémité distale, le dispositif peut être coiffé par un bouchon amovible. Hormis les différences mentionnées, ce mode de réalisation peut présenter les mêmes caractéristiques que celles décrites en référence aux figures 1 à 6.

### REFERENCES

- 1.: Conduit
- 2.: Plateau
- 3.: Ouverture
- 4.: Corps
- 5.: Paroi interne
- 6.: Deuxième canal
- 7.: Paroi externe
- 8.: Portion cylindrique
- 9.: Portion conique
- 10.: Premier canal
- 11.: Axe
- 12.: Portion cylindrique
- 13.: Portion conique
- 14.: Portion cylindre distale
- 15.: Embouchure
- 16.: Filetage
- 17.: Zone de raccordement
- 18.: Butée
- 19.: Joint
- 20.: Membrane
- 21.: Zone périphérique
- 22.: Zone mobile
- 23.: Trou
- 24.: Zone de confluence
- 25.: Sortie
- 26.: Contenant
- 27.: Zone périphérique
- 28.: Butée
- 29.: Bordure extérieure
- 30.: Rainure
- 31.: Surface amont
- 32.: Surface aval

## Revendications

1. Dispositif de délivrance d'un produit par pulvérisation, comportant un premier canal (10) de circulation du produit vers une sortie (25) du premier canal (10) et un deuxième canal (6) de circulation d'un gaz destiné à produire une dépression à la sortie (25) du premier canal (10) pour une aspiration du produit par effet Venturi, et comportant une membrane (20) configurée pour obstruer le deuxième canal (6) dans une première position et pour ouvrir le deuxième canal (6) dans une deuxième position, **caractérisé par le fait qu'**il comporte un plateau (2) s'étendant transversalement dans le deuxième canal (6) et configuré pour s'appliquer sur une face amont de ladite membrane (20) dans la première position, le plateau (2) comportant au moins une ouverture de passage de gaz obstruée par la membrane dans la première position et non obstruée par la membrane dans la deuxième position.

2. Dispositif selon la revendication précédente dans lequel le premier canal (10) et le deuxième canal (6) ont des directions moyennes parallèles au niveau de la sortie (25) du premier canal (10), les directions moyennes étant de préférence confondues.

3. Dispositif selon la revendication précédente, dans lequel le premier canal (10) est défini par la paroi interne d'un conduit (1) entouré par le deuxième canal (6).

4. Dispositif selon la revendication précédente dans lequel le premier canal (10) a une section transversale circulaire et le deuxième canal (6) a une section transversale annulaire.

5. Dispositif selon l'une des deux revendications précédentes, dans lequel ledit deuxième canal (6) est défini par une paroi externe (7) du conduit (1) d'une part et par une paroi interne (5) d'un corps (4) renfermant le conduit (1).

6. Dispositif selon la revendication précédente, dans lequel le plateau (2) s'étend entre la paroi externe (7) du conduit (1) vers la paroi interne (5) du corps (4).

7. Dispositif selon la revendication précédente, dans lequel la membrane (20) est solidaire par une bordure extérieure, de la paroi interne (5) du corps (4) et dans lequel la membrane (20) comporte une ouverture traversée par le conduit (1).

8. Dispositif selon la revendication précédente, dans lequel le corps (4) comporte sur sa paroi interne (5), une butée (18) configurée pour recevoir en appui une surface aval d'une zone périphérique (21) de la membrane (20), et de préférence dans lequel la paroi interne (5) comporte une portion filetée en amont de la butée (18).

9. Dispositif selon l'une des revendications 1 à 7, dans lequel la membrane (20) est solidaire de la paroi externe (7) du conduit (1) et dans lequel la membrane (20) comporte une bordure extérieure mobile entre la première position et la deuxième position.

10. Dispositif selon la revendication précédente, dans lequel la paroi externe (7) du conduit (1) comporte une rainure (30) dans laquelle est montée la bordure d'une ouverture de la membrane (20).

11. Dispositif selon l'une des deux revendications précédente, dans lequel le corps (4) comporte sur sa paroi interne (5), une butée (28) configurée pour recevoir en appui une surface aval d'une zone périphérique (27) du plateau (2), et de préférence dans lequel la paroi interne (5) comporte une portion filetée en amont de la butée (28).

12. Dispositif selon l'une des revendications précédentes, dans lequel le conduit (1) et le plateau (2) sont monoblocs.

13. Dispositif selon l'une des revendications précédentes dans lequel la section transversale du deuxième canal (6) est dégressive en direction de la sortie (25) et dans lequel dans lequel le deuxième canal (6) comporte une zone dont la section longitudinale est de forme conique dégressive en direction de la sortie (25), et de préférence dans lequel ladite zone se poursuit en aval de la sortie (25).

14. Pulvérisateur comportant un contenant apte à recevoir un produit à pulvériser, un dispositif selon l'un des revendications précédentes et une canule plongeant dans le contenant (26) et raccordée au premier canal (10), et de préférence dans lequel le contenant (26) comporte au moins une portion de paroi souple configurée pour appliquer une surpression au contenant lors d'un appui d'un utilisateur sur ladite portion.

15. Utilisation d'un pulvérisateur selon la revendication précédente pour la pulvérisation d'un produit sous forme de fluide.

## Patentansprüche

1. Vorrichtung zur Ausgabe eines Produkts durch Zerstäuben, umfassend einen ersten Kanal (10) zum Durchleiten des Produkts zu einem Ausgang (25) des ersten Kanals (10) hin, und einen zweiten Kanal (6) zum Durchleiten eines Gases, das dazu vorgesehen ist, am Ausgang (25) des ersten Kanals (10) einen Unterdruck für ein Ansaugen des Produkts durch Venturi-Effekt zu erzeugen, und umfassend eine Membran (20), die dafür ausgebildet ist, in einer ersten Stellung den zweiten Kanal (6) zu verschließen, und dafür, in einer zweiten Stellung den zweiten Kanal (6) zu öffnen, **gekennzeichnet durch** die Tatsache, dass sie eine Platte (2) umfasst, die sich quer im zweiten Kanal (6) erstreckt und dafür ausgebildet ist, sich in der ersten Stellung an eine stromaufwärtige Seite der Membran (20) anzulegen, wobei die Platte (2) mindestens eine Gasdurchlassöffnung umfasst, die in der ersten Stellung von der Membran verschlossen wird, und in der zweiten Stellung nicht von der Membran verschlossen wird.

2. Vorrichtung nach dem vorstehenden Anspruch, wobei der erste Kanal (10) und der zweite Kanal (6) mittlere Richtungen aufweisen, die im Bereich des Ausgangs (25) des ersten Kanals (10) parallel sind, wobei die mittleren Richtungen vorzugsweise zusammenfallen.

3. Vorrichtung nach dem vorstehenden Anspruch, wobei der erste Kanal (10) von der Innenwand einer Leitung (1) definiert wird, die vom zweiten Kanal (6) umgeben ist.

4. Vorrichtung nach dem vorstehenden Anspruch, wobei der erste Kanal (10) einen kreisförmigen Querschnitt aufweist, und der zweite Kanal (6) einen ringförmigen Querschnitt aufweist.

5. Vorrichtung nach einem der zwei vorstehenden Ansprüche, wobei der zweite Kanal (6) einerseits von einer Außenwand (7) der Leitung (1), und von einer Innenwand (5) eines die Leitung (1) umschließenden Körpers (4) definiert wird.

6. Vorrichtung nach dem vorstehenden Anspruch, wobei sich die Platte (2) zwischen der Außenwand (7) der Leitung (1) zur Innenwand (5) des Körpers (4) hin erstreckt.

7. Vorrichtung nach dem vorstehenden Anspruch, wobei die Membran (20) über einen Außenrand fest mit der Innenwand (5) des Körpers (4) verbunden ist, und wobei die Membran (20) eine Öffnung umfasst, die von der Leitung (1) durchquert wird.

8. Vorrichtung nach dem vorstehenden Anspruch, wobei der Körper (4) an seiner Innenwand (5) einen Anschlag (18) umfasst, der dafür ausgebildet ist, eine stromabwärtige Fläche eines Umfangsbereichs (21) der Membran (20) unter Andruck aufzunehmen, und wobei die Innenwand (5) vorzugsweise stromaufwärts des Anschlags (18) einen Gewindeabschnitt umfasst.

9. Vorrichtung nach einem der Ansprüche 1 bis 7, wobei die Membran (20) fest mit der Außenwand (7) der Leitung (1) verbunden ist, und wobei die Membran (20) einen Außenrand umfasst, der zwischen der ersten Stellung und der zweiten Stellung beweglich ist.

10. Vorrichtung nach dem vorstehenden Anspruch, wobei die Außenwand (7) der Leitung (1) einen Schlitz (30) umfasst, in dem der Rand einer Öffnung der Membran (20) montiert ist.

11. Vorrichtung nach einem der zwei vorstehenden Ansprüche, wobei der Körper (4) an seiner Innenwand (5) einen Anschlag (28) umfasst, der dafür ausgebildet ist, eine stromabwärtige Fläche einen Umfangsbereich (27) der Platte (2) unter Andruck aufzunehmen, und wobei die Innenwand (5) vorzugsweise stromaufwärts des Anschlags (28) einen Gewindeabschnitt umfasst.

12. Vorrichtung nach einem der vorstehenden Ansprüche, wobei die Leitung (1) und die Platte (2) einteilig sind.

13. Vorrichtung nach einem der vorstehenden Ansprüche, wobei der Querschnitt des zweiten Kanals (6) in Richtung des Ausgangs (25) abnimmt, und wobei wobei der zweite Kanal (6) einen Bereich umfasst, dessen Längsschnitt von in Richtung des Ausgangs (25) abnehmender Kegelform ist, und wobei sich der Bereich vorzugsweise stromabwärts des Ausgangs (25) fortsetzt.

14. Zerstäuber, umfassend einen Behälter, der dazu in der Lage ist, ein zu zerstäubendes Produkt aufzunehmen, eine Vorrichtung nach einem der vorstehenden Ansprüche und eine Kanüle, die in den Behälter (26) eintaucht und mit dem ersten Kanal (10) verbunden ist, und wobei der Behälter (26) vorzugsweise mindestens einen nachgiebigen Wandabschnitt umfasst, der dafür ausgebildet ist, bei einem Druck eines Benutzers auf den Abschnitt einen Überdruck auf den Behälter aufzubringen.

15. Verwendung eines Zerstäubers nach dem vorstehenden Anspruch zum Zerstäuben eines Produkts in Fluidform.

## Claims

1. Device for delivering a product by spraying, comprising a first channel (10) for circulating the product towards an outlet (25) of the first channel (10) and a second channel (6) for circulating a gas configured to produce a depression at the outlet (25) of the first channel (10) to suction the product by Venturi effect, and comprising a membrane (20) configured to block the second channel (6) in a first position and to open the second channel (6) in a second position, **characterised by** the fact that it comprises a plate (2) extending transversally in the second channel (6) and configured to be applied on an upstream face of said membrane (20) in the first position, the plate (2) comprising at least one opening for passing gas, blocked by the membrane in the first position and not blocked by the membrane in the second position.

2. Device according to the preceding claim, wherein the first channel (10) and the second channel (6) have average directions parallel to the outlet (25) of the first channel (10), the average directions being preferably combined.

3. Device according to the preceding claim, wherein the first channel (10) is defined by the inner wall of a duct (1) surrounded by the second channel (6).

4. Device according to the preceding claim, wherein the first channel (10) has a circular cross-section and the second channel (6) has an annular cross-section.

5. Device according to one of the two preceding claims, wherein said second channel (6) is defined by an outer wall (7) of the duct (1) on the one hand, and by an inner wall (5) of a body (4) containing the duct (1).

6. Device according to the preceding claim, wherein the plate (2) extends between the outer wall (7) of the duct (1) towards the inner wall (5) of the body (4).

7. Device according to the preceding claim, wherein the membrane (20) is secured by an outer edge, of the inner wall (5) of the body (4) and wherein the membrane (20) comprises an opening passed through by the duct (1).

8. Device according to the preceding claim, wherein the body (4) comprises on the inner wall (5) thereof, a stop (18) configured to bear against a downstream surface of a peripheral zone (21) of the membrane (20), and preferably wherein the inner wall (5) comprises a tapered portion upstream of the stop (18).

9. Device according to one of claims 1 to 7, wherein the membrane (20) is secured to the outer wall (7) of the duct (1) and wherein the membrane (20) comprises a mobile outer edge between the first position and the second position.

10. Device according to the preceding claim, wherein the outer wall (7) of the duct (1) comprises a groove (30) wherein is mounted the edge of an opening of the membrane (20).

11. Device according to one of the two preceding claims, wherein the body (4) comprises on the inner wall (5) thereof, a stop (28) configured to bear against a downstream surface of a peripheral zone (27) of the plate (2), and preferably wherein the inner wall (5) comprises a tapered portion upstream of the stop (28).

12. Device according to one of the preceding claims, wherein the duct (1) and the plate (2) are one-piece.

13. Device according to one of the preceding claims, wherein the cross-section of the second channel (6) is degressive in the direction of the outlet (25) and wherein the second channel (6) comprises a zone of which the longitudinal section is of conic shape, degressive in the direction of the outlet (25), and preferably wherein said zone is followed downstream of the outlet (25).

14. Sprayer comprising a container capable of receiving a product to spray, a device according to one of the preceding claims and a cannula immersed in the container (26) and connected to the first channel (10), and preferably wherein the container (26) comprises at least one portion of flexible wall, configured to apply an overpressure to the container when a user press against said portion.

15. Use of a sprayer according to the preceding claim, for spraying a product in the form of fluid.
